# EUROPEAN PATENT SPECIFICATION

(11) **EP 1 843 721 B1**
(45) Date of publication and mention of the grant of the patent: **26.11.2014**
(21) Application number: 05855685.3
(22) Date of filing: 27.12.2005
(51) Int. Cl.: A61F 2/06

(54) **LOW PROFILE, DURABLE, REINFORCED ePTFE COMPOSITE GRAFT**
HALTBARE VERSTÄRKTE EPTFE-KOMPOSITPROTHESE MIT NIEDRIGEM PROFIL
GREFFON COMPOSITE ePTFE RENFORCÉ, DURABLE, PLAT

(30) Priority: 29.12.2004 US 25571
(43) Date of publication of application: 17.10.2007
(73) Proprietor: Boston Scientific Limited, Hamilton HM11 (BM)
(72) Inventor: SOWINSKI, Krzystof, Wallington, NJ 07057 (US); DONG, Jerry, Oakland, NJ 07436 (US)
(74) Representative: Vossius & Partner
(86) International application number: PCT/US2005/047168
(87) International publication number: WO 2006/071909

(56) References cited:
- US-A- 4 857 069
- US-A1- 2003 004 559
- US-B1- 6 605 119

## Description

The present invention relates generally to low profile, reinforced PTFE or ePTFE composite graft. More particularly, the present invention relates to a thermally laminated PTFE or ePTFE composite graft.

Implantable prostheses are commonly used in medical applications. One of the more common prosthetic structures is a tubular prosthesis which may be used as a vascular graft to replace or repair damaged or diseased blood vessels.

One form of a conventional tubular prosthesis specifically used for vascular grafts includes a textile tubular structure formed by weaving, knitting, braiding or any non-woven textile technique processing synthetic fibers into a tubular configuration. Such conventional textile prostheses are often thick walled tubular vessels having wall thicknesses that exceed one millimeter, which limits intraluminal delivery due to the high profile of the graft.

It is also well known to form a nontextile prosthesis, especially a tubular graft, from polymers such as polytetrafluoroethylene (PTFE). Such a nontextile tubular graft may be formed by stretching and expanding PTFE into a structure referred to as expanded polytetrafluoroethylene (ePTFE). Tubes formed of ePTFE exhibit certain beneficial properties as compared with textile prostheses. The expanded PTFE tube has a unique structure defined by nodes interconnected by fibrils. The node and fibril structure defines micropores which facilitate a desired degree of tissue ingrowth while remaining substantially fluid-tight. Tubes of ePTFE may be formed to be exceptionally thin and yet exhibit the requisite strength necessary to serve in the repair or replacement of a body lumen. The thinness of the ePTFE tube facilitates ease of implantation and deployment with minimal adverse impact on the body. Such thinness, however, may result in a decrease in radial tensile strength, radial burst strength or suture retention strength.

WO 03/037396 A1 relates to a green fluoropolymer tube and an implantable tubular prosthesis. The prosthesis includes two sheaths and a distensible member interposed therebetween. The prosthesis formed with the distensible member interposed between the sheaths will be formed with a thickness in the range of 50-400 µm, although for certain applications the prosthesis may have a thickness as large as 1,000 µm.

WO 95/05555 relates to a thin-wall tube comprising a tube having an exterior surface, a luminal surface, a wall thickness of less than about 0.25 mm, and a longitudinal axis, said tubing comprise at least one first layer and at least one second layer of porous expanded PTFE film wherein in the porous expanded PTFE film has a microstructure having fibrils oriented substantially parallel to each other and wherein the fibrils of the first layer of porous expanded PTFE film are oriented substantially perpendicular to the fibrils of the second layer of porous expanded PTFE film. According to an example the wall thickness of the tube was determined to be 0.06 mm.

It is therefore desirable to provide an implantable prosthesis, preferably in the form of a tubular vascular prosthesis, which achieves many of the above-stated benefits, such as low profile, without the resultant disadvantages associated therewith.

The present invention is defined by the features of the claims.

In one aspect a low profile, durable, reinforced composite implantable graft is provided. The composite graft includes (a) a first seamless tubular layer comprising biocompatible first polymeric material and having a luminal surface and an exterior surface; (b) a biocompatible reinforcing member arranged in a pattern to define a second tubular layer, the second tubular layer being disposed over the exterior surface of the first tubular layer; and (c) a third seamless tubular layer comprising biocompatible second polymeric material and having a luminal surface and an exterior surface, the luminal surface of the third tubular layer being securably disposed over the second tubular and over the first tubular layer; wherein the tubular layers define a wall of the graft, the wall having a wall thickness of less than 0.05 mm.

In this aspect of the present invention, the first polymeric material and the second polymeric materials may be the same polymeric material or the same class of polymeric material.

The biocompatible reinforcing members may comprise yarns. The yarns may be formed in a textile pattern, such as a braided textile pattern, a woven textile pattern, a knitted textile pattern, or combinations thereof. Further, the yarns may be helically wrapped over the luminal layer to provide for the reinforcing layer. Moreover, the biocompatible reinforcing members may comprise helically wrapped tape, such as polymeric tape.

The first and the second polymeric materials may be selected from the group consisting of polytetrafluoroethylene, expanded polytetrafluoroethylene and combinations thereof. The graft layers may be laminated together without the presence of an adhesive. The reinforcing members comprise polymeric, such as polytetrafluoroethylene yarns or metallic yarns. The reinforcing members may also be monofilament strands or multifilament yarns.

Desirably, the first layer is an extruded tube and the third layer is an extruded tube. The graft may be a self-supporting graft. The graft may also be crimped.

A method of forming a composite graft includes the steps of (a) providing an elongate tubular mandrel; (b) placing a first seamless tubular layer over the mandrel, the first layer comprising biocompatible first polymeric material and having a luminal surface and an exterior surface; (c) providing reinforcing members over the exterior surface, the yarns arranged in a pattern to define a second tubular layer; (d) providing a third seamless tubular layer over the yarns, the third layer comprising biocompatible second polymeric material and having a luminal surface and an exterior surface, the luminal surface being securably disposed over the reinforcing members and over the first tubular layer; (e) heat laminating the layers together to form a graft wall having a wall thickness of less than 0.05 mm. The step of providing the reinforcing members may be selected from the group consisting of braiding, knitting, weaving, helically winding and combinations thereof. Desirably, the step of heat laminating the layers is done in the absence of an adhesive.
FIG. 1 is a perspective view of a composite graft of the present invention.
FIG. 2 is a cross-sectional view of the composite-graft of FIG. 1 taken along the 2-2 axis.
FIG. 3 is a partial cutaway, perspective view of the composite graft of FIG. 1.
FIG. 4 is a schematic of a diamond braid useful in the present invention.
FIG. 5 is a schematic of a regular braid useful in the present invention.
FIG. 6 is a schematic of a Hercules braid useful in the present invention.
FIG. 7 is a schematic of a regular weave useful in the present invention.
FIG. 8 is a schematic of a knit useful in the present invention.
FIG. 9 depicts a helically wound yarn over a tubular graft layer.
FIG. 10 depicts a helically wound tape over a tubular graft layer.

### DETAILED DESCRIPTION OF THE PREFERRED EMBODIMENT:

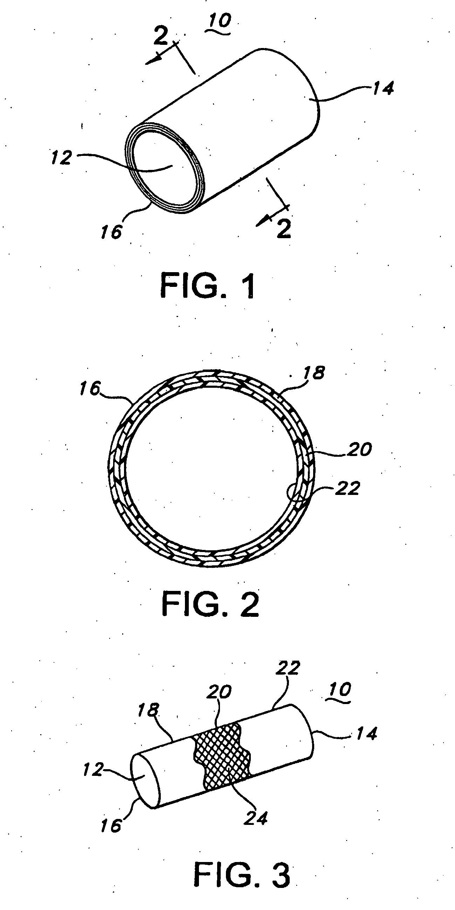

FIG. 1 is a perspective view of a composite graft 10 of the present invention. The graft 10 is an elongate hollow, tubular device having opposed open ends 12,14 and a graft wall 16. The thickness of the graft wall 16 is thin to provide a low profile to the composite graft 10. The thickness of the graft wall 16 is less than 0.05 mm.
FIG. 2 is a cross-sectional view of the graft **10** taken along the 2-2 axis of FIG. 1. As depicted in FIG. 2, graft wall **16** of the graft **10** includes an exterior portion **18,** a middle reinforcing portion **20** and a luminal or interior portion **22,** interrelated as shown. The exterior and luminal portions 1**8, 22** may be seamless tubular structures, such as thinly extruded tubes. Individually, the exterior and luminal portions **18, 22** may be as thin as **10** µm. Useful individual wall thicknesses include those from about 10 µm to less than 50 µm, more desirably from about 25 µm to less than 50 µm. Methods for producing thinly extruded tubes are described in U.S. Patent Application Publication Nos. 2003/0082323 A1 and 2003/0082324 A1.
FIG. 3 is a partial cutaway, perspective view of the graft **10** of the present invention further illustrating the exterior portion **18,** the middle reinforcing portion **20** and the luminal or interior portion **22.** In FIG. 3, the middle reinforcing portion is depicted as a braided portion of elongate members **24,** such as yarns. The present invention, however, is not so limited. For example, thin tapes, stands and the like may suitably be used with the practice of the present invention.

When the elongate members **24** are yarns, the yarns are desirably made from a textile material. The textile material may be formed from synthetic yarns that may be flat, shaped, twisted, textured, pre-shrunk or un-shrunk. Synthetic biocompatible yarns suitable for use in the present invention include, but are not limited to, polyesters, including polyethylene terephthalate (PET) polyesters, polypropylenes, polyethylenes, polyurethanes, polyolefins, polyvinyls, polymethylacetates, polyamides, naphthalane dicarboxylene derivatives, natural silk and polytetrafluoroethylenes. Moreover, at least one of the synthetic yarns may be a metallic yarn or a glass or ceramic yarn or fiber. Useful metallic yarns include those yarns made from or having stainless steel, platinum, gold, titanium, tantalum and Ni-Co-Cr-based alloy. The yarns may further comprise carbon, glass or ceramic fibers. Preferably, the yarns are made from thermoplastic materials including, but not limited to, polyesters, polypropylenes, polyethylenes, polyurethanes, polynaphthalenes, polytetrafluoroethylenes and the like. The yarns may be of the multifilament, monofilament or spun types. As is well known, the type and denier of the yarn chosen may be selected in a manner which forms a prosthesis and, more particularly, a vascular structure have desirable properties.

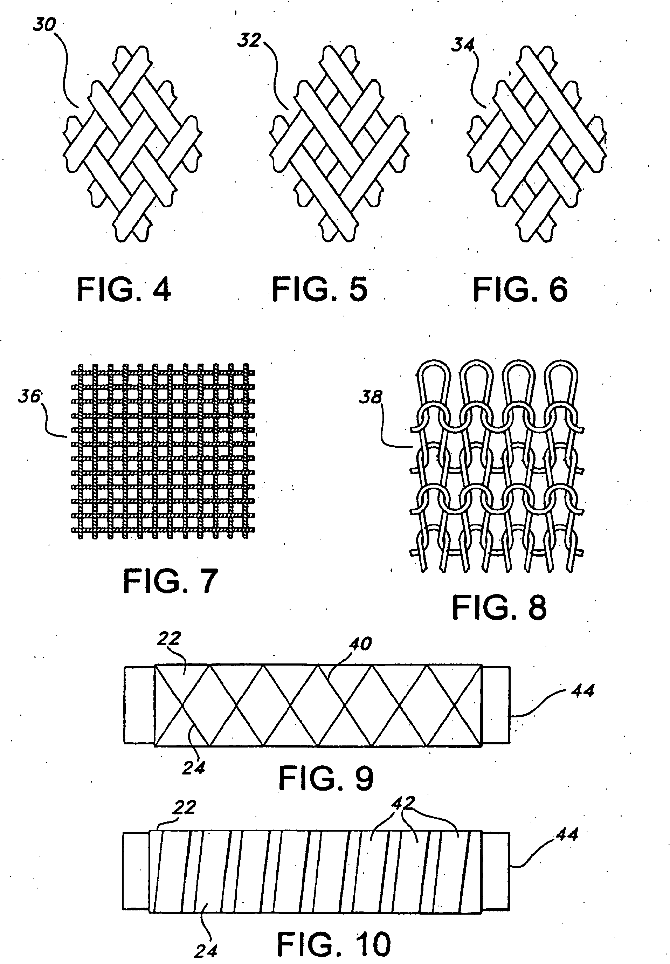

As depicted in FIG. 4-6, braiding of yarns includes the interlacing of at least two yarn systems such that the paths of the yarns are diagonal to the fabric delivery direction, forming a tubular structure. Useful braids include, but are not limited to, a diamond braid **30** having a 1/1 intersection repeat as depicted in FIG. 4, a regular braid **32** having a 2/2 intersection repeat as depicted in FIG. 4, or a Hercules braid **34** having a 3/3 intersection repeat as depicted in FIG. 4. U.S. Patent No. 5,653,746, further describes such braids. Moreover, a triaxial braid may also be used. A triaxial braid has at least one yarn that typically runs in the longitudinal direction or axial direction of the textile portion to limit yarn movement. The axial or longitudinal yarn is not interlaced or interwound with the other braid yarns, but is trapped between the different sets of yarns in the braided structure. Moreover, an interlocking three-dimensional braided structure or a multi-layered braided structure is also useful. A multi-layered braided structure is defined as a structure formed by braiding wherein the structure has a plurality of distinct and discrete layers.

Braiding machines, including circular braiding machines that form a braided textile over a mandrel, are useful with the practice of the present invention. An example of such a braiding machine is described in U.S. Patent No. 6,652,571. A braiding machine capable of forming the interlocked three-dimensional braid used to form the textile tube of the present invention is described in International Patent Publication No. WO 91/10766.

Generally, a braided structure is formed having a braid angle from about 30° to about 90° with respect to the longitudinal axis of the braided structure, desirably about 54.5° to about 75°. The yarns of the braid tend to seek equilibrium at a braid angle of about 54.5°, which is a neutral angle for tubular vessels under pressure. Thus, when the braid angle is larger than the neutral angle, when pressure is exerted from within, for example due to fluid flow, the yarns will tend to scissor and decrease the braid angle thereby elongating or stretching the braided structure in order to reach the neutral angle.

Useful weaves include, but are not limited to, a plain or regular weave 36 as depicted in FIG. 7, a basket weave, a twill weave, a satin weave, a velour weave and the like. U.S. Patent No. 5,653,746, further describes such weaves. The weave may be a circular weave or may be a flat woven tubular weave. Both flat weaving machines and circular weaving machines are known in the art. Circular weaving is a textile method where a tubular textile may be woven directly on a mandrel. A useful circular weaving machine in described in U.S. Patent No. 3,719,210.

Knitting involves the interlooping of one yarn system into vertical columns and horizontal rows of loops called wales and courses, respectively, with fabric coming out of the machine in the wale direction. Useful knits include, but are not limited to a high stretch knit, a locknit knit, which is also referred to as tricot or jersey knit (e.g., knit 38 as depicted in FIG. 8), reverse locknit knits, sharkskin knits, queenscord knits and velour knits. U.S. Patent No. 5,653,746, further describes useful knits. Useful high stretch, warp-knitted patterns include those with multiple patterns of diagonally shifting yarns, such as certain modified atlas knits which are described in U.S. Patent No. 6,540,773. Other useful high-stretch, warp knitted patterns include certain patterns with multiple needle underlap and one needle overlap, such as those patterns described in U.S. Patent No. 6,554,855 and U.S. Patent Application Publication No. 2003/0204241 A1. The knit may be a circular knit or may be a flat knitted tubular knit. Both flat knitting machines and circular knitting machines are known in the art. Circular knitting is a textile method where a tubular textile may be knitted directly on a mandrel. A useful circular weaving machine in described in U.S. Patent No. 6,640,590.

As depicted in FIG. 9, elongate member 24 may be a yarn 40 that may be helically wound over luminal layer 22. The elongate member may be wrapped in both longitudinal directions as illustrated in FIG. 9, or the elongate member may be wrapped in a single direction. The number and type of windings depend, in part, upon the properties of the elongate members 24 and the desired properties of the composite graft 10. The density of the wrap, i.e., the spacing of successive helical windings, may be varied so as to vary the coverage of the yarn over the external surface. The wrapping of yarns or strands may be varied from helical windings that are significantly spaced apart to tightly spaced windings. The number of wraps per inch may vary from about 5 to about 50, desirably from about 10 to about 30. Further the yarns may be splayed, i.e., flattened, especially in the case of multifilament yarns, to lower the profile of the reinforcing layer 20. When the elongate member 24 is a tape 42 as depicted in FIG. 10, successive helical windings be spaced apart as illustrated or may overlap (not shown). Further, as only one helical winding direction of tape 42 is depicted in FIG. 10, the present invention is not so limited and two or multi-directional winding patterns may suitably be used.

Desirably, the exterior layer 18 and the interior layer 22 are formed from polytetrafluoroethylene (PTFE) and/or expanded polytetrafluoroethylene (ePTFE). An ePTFE layer may be produced from the expansion of PTFE formed in a paste extrusion process. The PTFE extrusion may be expanded and sintered in a manner to form ePTFE having a microporous structure defined by nodes interconnected by elongate fibrils. The distance between the nodes, referred to as the internodal distance, may be varied by the parameters employed during the expansion and sintering process. The resulting process of expansion and sintering yields pores within the structure of the ePTFE layer. The sizes of the pores are defined by the internodal distance of the ePTFE layer. Additional details for extruding thin-walled PTFE and ePTFE seamless tubes is disclosed in U.S. Patent Application Publication 2003/0082324 A1.

The exterior layer **18** and the interior layer **22** and/or the reintorcing layer **20** may be adhesively bonded to form a composite prosthesis. The bonding agent may include various biocompatible, elastomeric bonding agents such as urethanes, styrene/isobutylene/styrene block copolymers (SIBS), silicones, and combinations thereof. Other similar materials are contemplated. Desirably, the bonding agent may include polycarbonate urethanes sold under the trade name CORETHANE^{®}. This urethane is provided as an adhesive solution with preferably 7.5% Corethane, 2.5 W30, in dimethylacetamide (DMAc) solvent. Additional details of suitable adhesives and methods for adhesively bonding graft layers are disclosed in U.S. Patent Application Publication No. 2003/0139806 A1.

Alternatively, the exterior layer **18** and the interior layer **22** and/or the reinforcing layer **20** may be thermally bonded to form a composite prosthesis. Desirably, the exterior layer **18** and the interior layer **22** and/or the reinforcing layer **20** are made from the same polymeric material, such as polytetrafluoroethylene, including expanded polytetrafluoroethylene, to facilitate the heat fusing of similar polymeric materials. Advantageously, the interior layer **22,** the reinforcing layer **20** and the exterior layer **18** are placed sequentially over a tubular mandrel **44.** A silicone tube may be placed over the composite graft components to apply a pressure from about 1 psig to about 10 psig, which facilitates the bonding process. The graft components may then be placed in an oven to thermally bond the components to one and the other. When the components' material is polytetrafluoroethylene, useful heating or laminating conditions include a temperature from about 300°C to about 400°C for a period of about 5 minutes to about 30 minutes. Other useful heating durations include from about 10 minutes to about 20 minutes, desirably about 15 minutes. Other useful temperatures include from about 330 °C to about 370°C, desirably, from about 340°C to about 360°C. Additional details of pressure lamination techniques for tubular grafts may be found in U.S. Patent No. 6,139,573 and U.S. Patent Application No. 10/741,209, filed December 19, 2003.

In one aspect of the present invention, a composite implantable graft is provided. The composite graft may include (a) a first seamless tubular layer comprising biocompatible first polymeric material and having a luminal surface and an exterior surface; (b) biocompatible reinforcing member arranged in a pattern to define a second tubular reinforcing layer, the second tubular layer being disposed over the exterior surface; and (c) a third seamless layer comprising biocompatible second polymeric material and having a luminal surface and an exterior surface, the luminal surface being securably disposed over the reinforcing members and over the first tubular layer; wherein the tubular layers define a wall of the graft, the wall having a wall thickness of less than 0.05 mm.

The graft may be crimped to provide kink resistance and or to provide the graft with longitudinal flexibility and or a self-supporting wall feature. The present invention, however, is not limited to crimping to provide such mechanical features, such as a self-supporting graft wall. For example, the reinforcing members may be metallic yarns or strands arranged in a pattern to provide the self-supporting feature of this aspect of the present invention. The reinforcing members may also comprise metallic yarns. The yarns may be monofilament strands or multifilament yarns.

Desirably, the first polymeric material and the second polymeric material are the same, for example polytetrafluoroethylene or expanded polytetrafluoroethylene. The biocompatible reinforcing members may include yarns. The yarns may be formed in a textile pattern, such as a braided textile pattern, a woven textile pattern, a knitted textile pattern, or combinations thereof. Further, the yarns may be arranged as a helical wrap of the yarns to provide the reinforcing layer. Moreover, the biocompatible reinforcing members may comprise a helically wrapped tape. The biocompatible reinforcing members comprise a third polymeric material which may be the same or different from the first and the second polymeric materials. Desirably, these polymeric materials are the same, for example, polytetrafluoroethylene, expanded polytetrafluoroethylene and combinations thereof. The layers may be laminated together without the presence of an adhesive.

The graft wall of the composite graft of the present invention may be substantially fluid tight.

Desirably, the first layer is an extruded tube. The third layer may also be an extruded tube.

In another aspect or the present invention, an ePTFE or PTFE composite implantable graft is provided. The graft comprises (a)a first seamless tubular layer consisting essentially of a biocompatible polymeric material selected from the group consisting of polytetrafluoroethylene, expanded polytetrafluoroethylene and combinations thereof and having a luminal surface and an exterior surface; (b) a biocompatible reinforcing layer comprising a thin, elongate member arranged in a noncontiguous pattern to define a noncontinuous second tubular layer, the elongate member consisting essentially of the biocompatible polymeric material, the second tubular layer being disposed over the exterior surface of the first tubular layer; and (c) a third seamless tubular layer consisting essentially of the biocompatible polymeric material and having a luminal surface and an exterior surface, the luminal surface of the third tubular layer being securably disposed over the second tubular layer and over the first tubular layer; wherein the layers are laminated together without the presence of an adhesive.

A method of forming the composite graft of the present invention includes the steps of (a) providing an elongate tubular mandrel; (b) placing a first seamless tubular layer over the mandrel, the first layer comprising biocompatible first polymeric material and having a luminal surface and an exterior surface; (c) providing reinforcing members over the exterior surface, the yarns arranged in a pattern to define a second tubular layer; (d) providing a third seamless tubular layer over the yarns, the third layer comprising biocompatible second polymeric material and having a luminal surface and an exterior surface, the luminal surface being securably disposed over the reinforcing members and over the first tubular layer; (e) heat laminating the layers together to form a graft wall having a wall thickness of less than 0.05 mm.

The step of providing the reinforcing members may be selected from the group consisting of braiding, knitting, weaving, helically winding and combinations thereof. Further, the step of heat laminating the layers is advantageously done in the absence of an adhesive.

With any embodiment of the graft 10 may be formed as a self-supporting prosthesis and usable to maintain patency of a bodily vessel, such as in the coronary vasculature, esophagus, trachea, colon, biliary tract, urinary tract, prostate, and brain. Also, stent-graft 10 may be treated with any of the following: anti-thrombogenic agents (such as heparin, heparin derivatives, urokinase, and PPack (dextrophenylalanine proline arginine chloromethylketone); anti-proliferative agents (such as enoxaprin, angiopeptin, or monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-miotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick antiplatelet peptides); vascular cell growth promotors (such as growth factor inhibitors, growth factor receptor antagonists, transcriptional activators, and translational promotors); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

Various changes to the foregoing described and shown structures will now be evident to those skilled in the art. Accordingly, the particularly disclosed scope of the invention is set forth in the following claims.

## Claims

1. A composite implantable graft (10) comprising:
a first seamless tubular layer (22) comprising biocompatible first polymeric material and having a luminal surface and an exterior surface;
a biocompatible reinforcing member (24) arranged in a pattern to define a second tubular layer (20), said second tubular layer (20) being disposed over said exterior surface of said first tubular layer (22); and
a third seamless tubular layer (18) comprising biocompatible second polymeric material and having a luminal surface and an exterior surface, said luminal surface of said third tubular layer (18) being securably disposed over said second tubular layer (20) and over said first tubular layer (22);
wherein said tubular layers (18, 20, 22) define a wall of said graft (10), said wall having a wall thickness of less than 0.05 mm.

2. A graft (10) according to claim 1, wherein the graft (10) is crimped.

3. A graft (10) according to any one of the preceding claims, wherein said first polymeric material and said second polymeric material are the same.

4. A graft (10) according to any of the preceding claims, wherein said biocompatible reinforcing member (24) comprises yarns (40).

5. A graft (10) according to claim 4, wherein said pattern is a textile pattern selected from the group consisting of a braided textile pattern (30, 32, 34), a woven textile pattern (36), a knitted textile pattern (38), or combinations thereof.

6. A graft (10) according to claim 5, wherein said pattern is a helical wrap of said yarns (40).

7. A graft (10) according to claims 1 through 3, wherein said biocompatible reinforcing member (24) comprises a helically wrapped tape (42).

8. A graft (10) according to any of the preceding claims, wherein said biocompatible reinforcing member (24) comprises a third polymeric material and further wherein said first, said second and said third polymeric materials are the same class of polymers.

9. A graft (10) according to claim 4, wherein said first and said second polymeric materials are selected from the group consisting of polytetrafluoroethylene, expanded polytetrafluoroethylene and combinations thereof.

10. A graft (10) according to any of the preceding claims, wherein said layers (18, 20, 22) are laminated together without the presence of an adhesive.

11. A graft (10) according to claim 1, wherein said reinforcing member (24) comprises metallic yarns.

12. A graft (10) according to claim 1, wherein said reinforcing member (24) comprises a monofilament strand.

13. A graft (10) according to any of the preceding claims, wherein said graft wall is substantially fluid tight.

14. A graft (10) according to any of the preceding claims, wherein said first layer (22) is an extruded tube.

15. A graft (10) according to any of the preceding claims, wherein said third layer (18) is an extruded tube.

16. A graft (10) according to any of the preceding claims, wherein said graft (10) is a self-supporting graft.

17. A graft (10) according to claim 16, wherein said graft wall is crimped.

18. A graft (10) according to claim 1, wherein said first seamless tubular layer (22) consists essentially of polytetrafluoroethylene, expanded polytetrafluoroethylene and combinations thereof, said reinforcing member (24) being arranged in a noncontiguous pattern, said reinforcing member (24) consisting essentially polytetrafluoroethylene, expanded polytetrafluoroethylene and combinations thereof; said third steamless tubular layer (18) consisting essentially of polytetrafluoroethylene, expanded polytetrafluoroethylene and combinations thereof; and wherein said layers (18, 20, 22) are laminated together with the presence of an adhesive.

19. A method of forming a composite graft (10) comprising:
providing an elongate tubular mandrel;
placing a first seamless tubular layer (22) over said mandrel, said first layer (22) comprising biocompatible first polymeric material and having a luminal surface and an exterior surface;
providing a reinforcing member (24) over said exterior surface, said member (24) arranged in a pattern to define a second tubular layer (20);
providing a third seamless tubular layer (18) over said member (24), said third layer (18) comprising biocompatible second polymeric material and having a luminal surface and an exterior surface, said luminal surface being securably disposed over said reinforcing member (24) and over said first tubular layer (22);
heat laminating said layer (18, 20, 22) together to form a graft wall having a wall thickness of less than 0.05 mm.

20. A method according to claim 19, wherein the step of providing said reinforcing member (24) is selected from the group consisting of braiding, knitting, weaving, helically winding and combinations thereof.

21. A method according to claims 19 through 20, wherein the step of heat laminating said layers (18, 20, 22) is done in the absence of an adhesive.

## Patentansprüche

1. Implantierbare Kompositprothese (10), die aufweist:
eine erste nahtlose röhrenförmige Schicht (22), die biokompatibles erstes Polymermaterial aufweist und eine Lumenfläche und eine Außenfläche hat;
ein biokompatibles Verstärkungsbauteil (24), das in einem solchen Muster angeordnet ist, dass eine zweite röhrenförmige Schicht (20) gebildet ist, wobei die zweite röhrenförmige Schicht (20) über der Außenfläche der ersten röhrenförmigen Schicht (22) angeordnet ist; und
eine dritte nahtlose röhrenförmige Schicht (18), die biokompatibles zweites Polymermaterial aufweist und eine Lumenfläche und eine Außenfläche hat, wobei die Lumenfläche der dritten röhrenförmigen Schicht (18) über der zweiten röhrenförmigen Schicht (20) und über der ersten röhrenförmigen Schicht (22) befestigbar angeordnet ist;
wobei die röhrenförmigen Schichten (18, 20, 22) eine Wand der Prothese (10) bilden, wobei die Wand eine Wanddicke von weniger als 0,05 mm hat.

2. Prothese (10) nach Anspruch 1, wobei die Prothese (10) gecrimpt ist.

3. Prothese (10) nach einem der vorstehenden Ansprüche, wobei das erste Polymermaterial und das zweite Polymermaterial gleich sind.

4. Prothese (10) nach einem der vorstehenden Ansprüche, wobei das biokompatible Verstärkungsbauteil (24) Garne (40) aufweist.

5. Prothese (10) nach Anspruch 4, wobei das Muster ein Textilmuster ist, das aus der Gruppe ausgewählt ist, die aus einem Flechttextilmuster (30, 32, 34), einem Webtextilmuster (36), einem Stricktextilmuster (38) oder deren Kombinationen besteht.

6. Prothese (10) nach Anspruch 5, wobei das Muster eine Spiralwicklung der Garne (40) ist.

7. Prothese (10) nach einem der Ansprüche 1 bis 3, wobei das biokompatible Verstärkungsbauteil (24) ein spiralförmig gewickeltes Band (42) aufweist.

8. Prothese (10) nach einem der vorstehenden Ansprüche, wobei das biokompatible Verstärkungsbauteil (24) ein drittes Polymermaterial aufweist und wobei ferner das erste, das zweite und das dritte Polymermaterial zur gleichen Klasse von Polymeren gehören.

9. Prothese (10) nach Anspruch 4, wobei das erste und das zweite Polymermaterial aus der Gruppe ausgewählt sind, die aus Polytetrafluorethylen, expandiertem Polytetrafluorethylen und deren Kombinationen besteht.

10. Prothese (10) nach einem der vorstehenden Ansprüche, wobei die Schichten (18, 20, 22) ohne vorhandenen Kleber gemeinsam laminiert sind.

11. Prothese (10) nach Anspruch 1, wobei das Verstärkungsbauteil (24) metallische Garne aufweist.

12. Prothese (10) nach Anspruch 1, wobei das Verstärkungsbauteil (24) einen Monofilstrang aufweist.

13. Prothese (10) nach einem der vorstehenden Ansprüche, wobei die Prothesenwand im Wesentlichen fluiddicht ist.

14. Prothese (10) nach einem der vorstehenden Ansprüche, wobei die erste Schicht (22) eine extrudierte Röhre ist.

15. Prothese (10) nach einem der vorstehenden Ansprüche, wobei die dritte Schicht (18) eine extrudierte Röhre ist.

16. Prothese (10) nach einem der vorstehenden Ansprüche, wobei die Prothese (10) eine selbsttragende Prothese ist.

17. Prothese (10) nach Anspruch 16, wobei die Prothesenwand gecrimpt ist.

18. Prothese (10) nach Anspruch 1, wobei die erste nahtlose röhrenförmige Schicht (22) im Wesentlichen aus Polytetrafluorethylen, expandiertem Polytetrafluorethylen und deren Kombinationen besteht; wobei das Verstärkungsbauteil (24) in einem nichtkontinuierlichen Muster angeordnet ist, das Verstärkungsbauteil (24) im Wesentlichen aus Polytetrafluorethylen, expandiertem Polytetrafluorethylen und deren Kombinationen besteht; die dritte nahtlose röhrenförmige Schicht (18) im Wesentlichen aus Polytetrafluorethylen, expandiertem Polytetrafluorethylen und deren Kombinationen besteht; und wobei die Schichten (18, 20, 22) ohne vorhandenen Kleber gemeinsam laminiert sind.

19. Verfahren zur Bildung einer Kompositprothese (10), das aufweist:
Bereitstellen eines länglichen röhrenförmigen Dorns;
Platzieren einer ersten nahtlosen röhrenförmigen Schicht (22) über dem Dorn, wobei die erste Schicht (22) biokompatibles erstes Polymermaterial aufweist und eine Lumenfläche und eine Außenfläche hat;
Bereitstellen eines Verstärkungsbauteils (24) über der Außenfläche, wobei das Bauteil (24) in einem solchen Muster angeordnet wird, dass eine zweite röhrenförmige Schicht (20) gebildet wird;
Bereitstellen einer dritten nahtlosen röhrenförmigen Schicht (18) über dem Bauteil (24), wobei die dritte Schicht (18) biokompatibles zweites Polymermaterial auf weist und eine Lumenfläche und eine Außenfläche hat, wobei die Lumenfläche über dem Verstärkungsbauteil (24) und über der ersten röhrenförmigen Schicht (22) befestigbar angeordnet wird;
gemeinsames Wärmelaminieren der Schichten (18, 20, 22), um eine Prothesenwand mit einer Wanddicke von weniger als 0,05 mm zu bilden.

20. Verfahren nach Ansprüchen 19 bis 20, wobei der Schritt des Bereitstellens des Verstärkungsbauteils (24) aus der Gruppe ausgewählt ist, die aus Flechten, Stricken, Weben, spiralförmigem Wickeln und deren Kombinationen besteht.

21. Verfahren nach Anspruch 19 bis 20, wobei der Schritt des Wärmelaminierens der Schichten (18, 20, 22) in Abwesenheit eines Klebers durchgeführt wird.

## Revendications

1. Greffe implantable composite (10) comprenant :
une première couche tubulaire sans soudure (22) comprenant un premier matériau polymère biocompatible et ayant une surface luminale et une surface extérieure ;
un membre de renfort biocompatible (24) disposé dans un motif pour définir une seconde couche tubulaire (20), ladite seconde couche tubulaire (20) étant disposée sur ladite surface extérieure de ladite première couche tubulaire (22) ; et
une troisième couche tubulaire sans soudure (18) comprenant un second matériau polymère biocompatible et ayant une surface luminale et une surface extérieure, ladite surface luminale de ladite troisième couche tubulaire (18) étant disposée de manière immobilisable sur ladite seconde couche tubulaire (20) et sur ladite première couche tubulaire (22) ;
où lesdites couches tubulaires (18, 20, 22) définissent une paroi de ladite greffe (10), ladite paroi ayant une épaisseur de paroi inférieure à 0,05 mm.

2. Greffe (10) selon la revendication 1, où la greffe (10) est ondulée.

3. Greffe (10) selon l'une quelconque des revendications précédentes, où ledit premier matériau polymère et ledit second matériau polymère sont les mêmes.

4. Greffe (10) selon l'une quelconque des revendications précédentes, où ledit membre de renfort biocompatible (24) comprend des fils (40).

5. Greffe (10) selon la revendication 4, où ledit motif est un motif textile choisi dans le groupe consistant en un motif textile tressé (30, 32, 34), un motif textile tissé (36), un motif textile tricoté (38), ou leurs combinaisons.

6. Greffe (10) selon la revendication 5, où ledit motif est un enroulement hélicoïdal desdits fils (40).

7. Greffe (10) selon les revendications 1 à 3, où ledit membre de renfort biocompatible (24) comprend un ruban enroulé en hélice (42).

8. Greffe (10) selon l'une quelconque des revendications précédentes, où ledit membre de renfort biocompatible (24) comprend un troisième matériau polymère et où, en outre, ledit premier, ledit second et ledit troisième matériau polymère sont la même classe de polymères.

9. Greffe (10) selon la revendication 4, où ledit premier et ledit second matériaux polymères sont choisis dans le groupe consistant en le polytétrafluoroéthylène, le polytétrafluoroéthylène expansé et leurs combinaisons.

10. Greffe (10) selon l'une quelconque des revendications précédentes, où lesdites couches (18, 20, 22) sont stratifiées ensemble sans la présence d'un adhésif.

11. Greffe (10) selon la revendication 1, où ledit membre de renfort (24) comprend des fils métalliques.

12. Greffe (10) selon la revendication 1, où ledit membre de renfort (24) comprend un câble monofilament.

13. Greffe (10) selon l'une quelconque des revendications précédentes, où ladite paroi de greffe est sensiblement étanche aux fluides.

14. Greffe (10) selon l'une quelconque des revendications précédentes, où ladite première couche (22) est un tube extrudé.

15. Greffe (10) selon l'une quelconque des revendications précédentes, où ladite troisième couche (18) est un tube extrudé.

16. Greffe (10) selon l'une quelconque des revendications précédentes, où ladite greffe (10) est une greffe autoporteuse.

17. Greffe (10) selon la revendication 16, où ladite paroi de greffe est ondulée.

18. Greffe (10) selon la revendication 1, où ladite première couche tubulaire sans soudure (22) consiste essentiellement en polytétrafluoroéthylène, polytétrafluoroéthylène expansé et leurs combinaisons ; ledit membre de renfort (24) étant disposé dans un motif non contigu, ledit membre de renfort (24) consistant essentiellement en polytétrafluoroéthylène, polytétrafluoroéthylène expansé et leurs combinaisons ; ladite troisième couche tubulaire sans soudure (18) consistant essentiellement en polytétrafluoroéthylène, polytétrafluoroéthylène expansé et leurs combinaisons ; et où lesdites couches (18, 20, 22) sont stratifiées ensemble sans la présence d'un adhésif.

19. Procédé de formation d'une greffe composite (10) comprenant :
la fourniture d'un mandrin tubulaire allongé ;
la mise en place d'une première couche tubulaire sans soudure (22) sur ledit mandrin, ladite première couche (22) comprenant un premier matériau polymère biocompatible et ayant une surface luminale et une surface extérieure ;
la fourniture d'un membre de renfort (24) sur ladite surface extérieure, ledit membre (24) disposé dans un motif pour définir une seconde couche tubulaire (20) ;
la fourniture d'une troisième couche tubulaire sans soudure (18) sur ledit membre (24), ladite troisième couche (18) comprenant un second matériau polymère biocompatible et ayant une surface luminale et une surface extérieure, ladite surface luminale étant disposée de manière immobilisable sur ledit membre de renfort (24) et sur ladite première couche tubulaire (22) ;
la stratification à la chaleur desdites couches (18, 20, 22) ensemble pour former une paroi de greffe ayant une épaisseur de paroi inférieure à 0,05 mm.

20. Procédé selon la revendication 19, où l'étape de fourniture dudit membre de renfort (24) est choisie dans le groupe consistant en le tressage, le tricotage, le tissage, l'enroulement en hélice et leurs combinaisons.

21. Procédé selon les revendications 19 à 20, où l'étape de stratification à la chaleur desdites couches (18, 20, 22) est accomplie en l'absence d'un adhésif.
